# EUROPEAN PATENT SPECIFICATION

(11) **EP 2 653 099 B1**
(45) Date of publication and mention of the grant of the patent: **16.12.2015**
(21) Application number: 11848367.6
(22) Date of filing: 20.09.2011
(51) Int. Cl.: A61B 5/151

(54) **LANCET DEVICE CAPABLE OF LANCET DETACHMENT**
ZUR ABLÖSUNG EINER LANZETTE GEEIGNETE LANZETTENVORRICHTUNG
DISPOSITIF À LANCETTE PERMETTANT UN DÉTACHEMENT DE LANCETTE

(30) Priority: 16.12.2010 KR 20100129246
(43) Date of publication of application: 23.10.2013
(73) Proprietor: i-Sens, Inc., Nowon-gu, Seoul 139-050 (KR)
(72) Inventor: CHA, Geun Sig, Seoul 120-100 (KR); NAM, Hakhyun, Seoul 142-063 (KR); CHA, Eun-Jong, Cheongju-si Chungcheongbuk-do 361-753 (KR); KIM, Kyung-Ah, Cheongju-si Chungcheongbuk-do 361-765 (KR)
(74) Representative: Modiano, Micaela Nadia
(86) International application number: PCT/KR2011/006935
(87) International publication number: WO 2012/081808

(56) References cited:
- EP-A1- 0 458 451
- KR-A- 20090 049 505
- KR-A- 20090 099 969
- KR-B1- 100 912 202
- US-A1- 2007 083 222

## Description

### Field of the Invention

The present invention relates to a lancet-separable lancet device. More particularly, the present invention relates to a lancet-separable lancet device which can minimize the danger of being pricked or touched by a lancet needle due to spring of a lancet when the lancet is separated.

### Description of the Related Art

In general, chronic diabetics have to measure the blood glucose level by performing a blood sugar test by themselves everyday at home and to perform disease control in order to keep a predetermined blood glucose level.

They have to collect blood for the blood glucose test, and this case, generally, they stick a disposable lancet into the skin of a portion, usually a finger, of their bodies, take and put a small amount of capillary blood onto a strip, and then measure the blood glucose level using a blood glucose meter with the strip mounted.

A lancing device is generally used as the device for taking blood.

The lancing device is composed of a lancet holder mounted with a disposable lancet, a cover that covers a lancet and has a hole through which only the tip of a needle protrudes to penetrate a skin, and a spring and a releasing member that provide a penetration force. The disposable lancet has lancet needle at one end of a lancet body and a protection cap is combined with the lancet needle.

According to the lancing devices having this configuration in the related art, a user removes the cover from a lancing device, mounts a disposable lancet onto the lancet holder, attaches the cover with the spring compressed, brings the lancet in close contact with a portion with many capillaries such as fingers, and then releases the disposable lancet by pulling a releasing switch, such that the lancet penetrates the skin.

Lancets are supposed to be disposable to prevent infection, because they penetrate the skin of a human.

Therefore, a subject should remove a lancet after using it with a lancing device.

However, a subject should remove a lancet by hand when separating a disposable lancet used for taking blood and the lancet is separated and springs by the internal elastic force, such that there is some possibility that the lancet pricks the skin of the subject or people around, which causes a serious problem in safety.

In particular, when an examiner (the third person) takes blood, the examiner's hand may be pricked or touched by the needle with the blood of the subject thereon when separating a disposable lancet from a lancing device, such that there is a problem in that viruses may be transmitted.

Examples of lancet devices with lancet removal devices are known from the following documents.

D1 discloses a lancet device including a body and a holding member movably mounted within the body and comprising a front end and a rear end. The front end is configured to receive a lancet having the lancet needle. A lancet ejector and/or a biasing member are utilized. The biasing member is one of arranged within the holding member and biasing a mechanism for setting a trigger.

D2 discloses a lancet which has a simple structure and which can be secured safely, in particular, after use, and a lancet device including a casing, and a puncture body which is accommodated in the casing so as to be movable in a puncturing direction. The lancet has a convex portion formed on an inner surface of the casing and a recessed portion (groove) formed on the puncture body, which form fitting for holding the puncture body so as to be immovable in the puncturing direction in the casing.

D3 discloses a lancet device which can adjust a puncture depth and a lancet needle separate therefrom by pressing a lancet when the lancet is separated.

### Disclosure

### Technical Problem

The present invention has been made in an effort to provide a lancet-separable lancet device having advantages of being able to minimize the danger of being pricked or touched by a lancet needle due to spring of a lancet when the lancet is separated.

### Technical Solution

An exemplary embodiment of the present invention provides a lancet-separable lancet device as defined in claim 1.

According to the present invention described above, since the lancet separation switch is used while the lancet that finished taking blood is separated from the lancet device, it is possible to minimize the possibility of sticking the skin of a subject or people around with the lancet needle, the subject can stably separate the lancet, and it is possible to minimize infection by viruses due to the reason that the examiner's fingers are pricked or touched by the lancet needle used for taking blood.

### Description of Drawings

FIG. 1 is a perspective view a lancet-separable lancet device according to an exemplary embodiment of the present invention.
FIG. 2 is an exploded perspective view the lancet-separable lancet device according to an exemplary embodiment of the present invention.
FIG. 3 is a cross-sectional view of the assembly of the parts shown in FIG. 1.
FIG. 4 is a status view for illustrating how to use a lancet separation mechanism of the lancet-separable lancet device according to an exemplary embodiment of the present invention.

### Best Mode

Hereinafter, exemplary embodiment of the present invention will be described in detail with reference to the accompanying drawings. First, when giving the components in the drawings reference numerals, it should be noted that the same reference numerals are given to reference numerals as same as possible even if they are shown in different drawings. Further, the in description of the present invention, the detailed description of related well-known configurations and functions is not provided, when it is determined as making the scope of the present invention unclear.

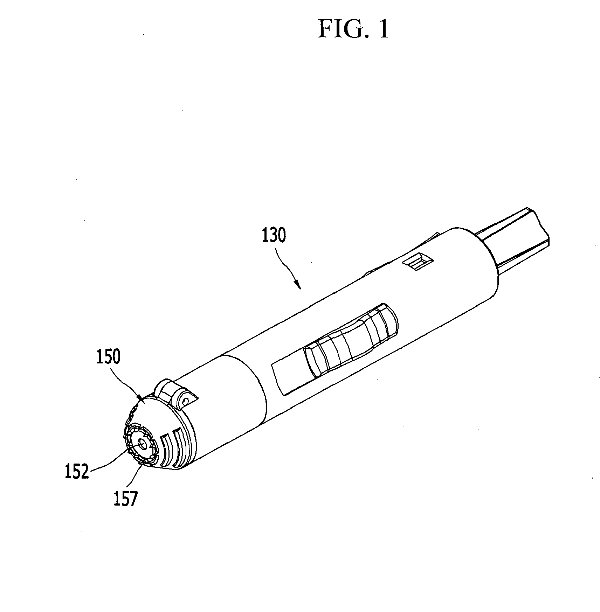

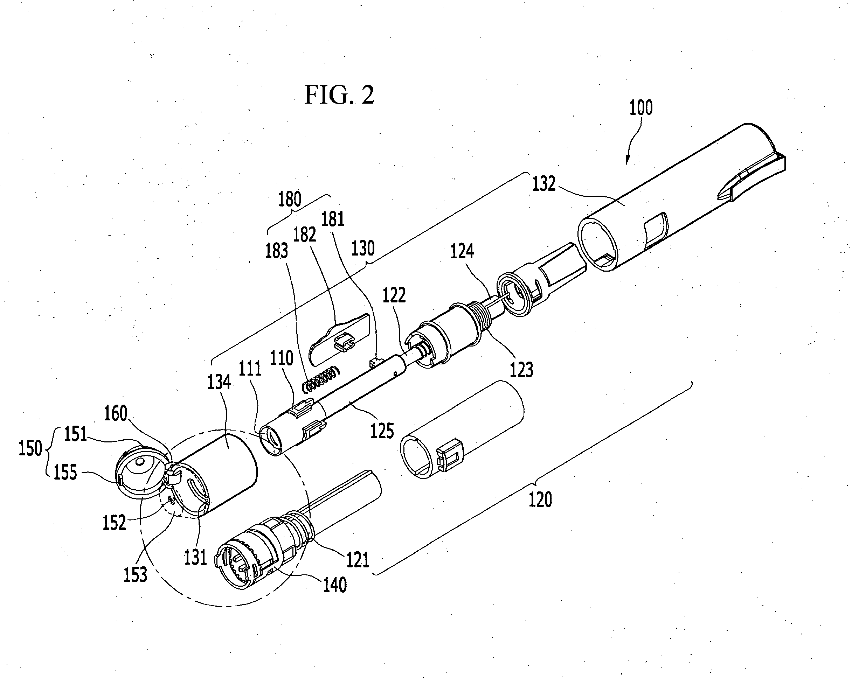

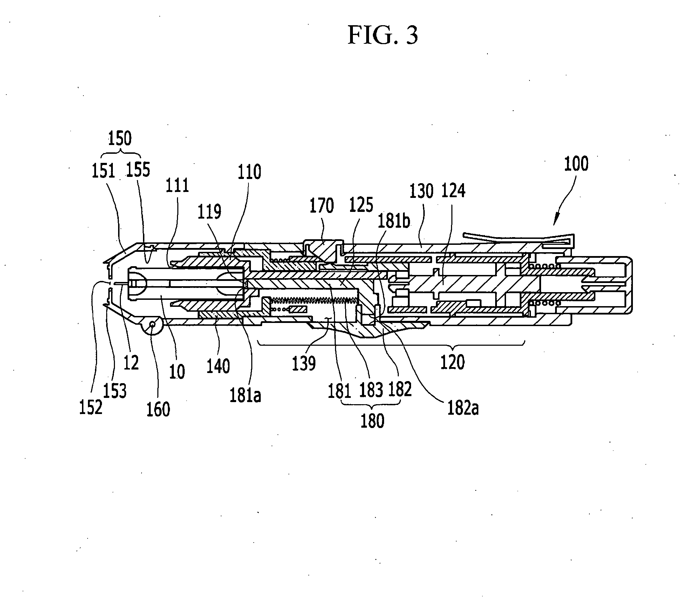

FIG. 1 is a perspective view a lancet-separable lancet device according to an exemplary embodiment of the present invention, FIG. 2 is an exploded perspective view the lancet-separable lancet device according to an exemplary embodiment of the present invention, and FIG. 3 is a cross-sectional view of the assembly of the parts shown in FIG. 1.

Referring to FIGS. 1 to 3, a lance-separable lancet device 100 according to an exemplary embodiment of the present invention largely includes a lancet holder 110, an operating mechanism 120, a housing 130, an operation switch 170, a depth adjusting member 140, a cover 150, a coupling portion 160, and a lancet separation mechanism 180.

The lancet holder 110 has a lancet seat 111 to mount a lancet 10 at one end and is disposed at a side in the housing 130.

The lancet 10, which is disposable, has a lancet needle 12 at one end of a lancet body 11 with a predetermined length and the lancet needle 12 is combined with a protection cap (not shown).

The protection cap is coupled to one end of the lancet needle 12 by insert injection, and according to this configuration, the protection cap can be separated from the lancet needle 12 even by a small force.

The operating mechanism 120 is disposed at the other end of the lancet holder 110, has a structure for loading and releasing the lancet holder 110 along a predetermined path, and for this operation, it includes a plurality of springs 121, 122, and 123, as shown in FIG. 1.

The housing 130, a cylinder sized to be portable by a user, receives the operating mechanism 120, with the lancet holder 110 coupled to one end, and the operation switch 170 operating with the operating mechanism 120 is disposed at a predetermined position.

The operation switch 170 is connected to the operating mechanism 120 and operates the operating mechanism 120 by pressing it.

When a user presses the operation switch 170, the springs 121, 122, and 123 are instantaneously compressed, and extend and hit a movable shaft 125 under which a shock transmission rod 124 is positioned, the movable shaft 125 hits the lancet holder 110 holding the lancet 10, and the lancet 10 protrudes outward.

The depth adjusting member 140 adjusts the penetration depth under the skin of the lancet needle 12.

The cover 150 covers one end of the housing 130 and has a through-hole 152 at the center through which the lancet needle 12 of the lancet 10 instantaneously protrudes and a plurality of protrusions 153 radially formed and arranged at predetermined angles on the front side.

The coupling portion 160 hinges one end of the housing 130 and the cover 150.

The lancet separation mechanism 180 is disposed at the other side of the lancet holder 110, and is inserted into the lancet holder 110 through a separation hole 119 of the lancet holder 110 and separates the lancet 10 from the lancet holder 110 by pressing the lancet 10. To this end, the lancet separation mechanism 180 includes an L-shaped bar 181 with an end 181 a inserted in the separation hole 119 of the lancet holder 110 and a lancet separation switch 182 that has a fitting recess 182a fitted to the other end 181 b with a step of the L-shaped bar 181 and is in close contact with the outer side of the housing 130.

The end 181 a of the L-shaped bar 181 is elongated in the longitudinal direction of the movable shaft 125 to be able to press the bottom of the lancet holder 110 through the separation hole 119 of the lancet holder 110 and the other end 181 b is formed perpendicularly from the end 181 a, has a step, and is fitted in the fitting recess 182a of the lancet separation switch 182.

The lancet separation switch 182 is fitted ir a coupling hole 139 formed on the outer side of the housing 130 and coupled to the other end 181 b of the bar 181, such that it inserts the bar 181 into the lancet holder 110 through the separation hole 119 of the lancet holder 110, when being moved in the longitudinal direction of the bar 181 by a user.

An elastic member 183 may be further provided so that the lancet separation switch 182 moved by a user can smoothly return to the initial position. The elastic member 183, which is a spring, has one end being in contact with the other end of the depth adjusting member 140 and the other end coupled to a side of the bar 181 or the lancet separation switch 182, such that it returns the bar 181 inserted in the separation hole 119 of the lancet holder 110 to the initial position.

In other words, the elastic member 183 is combined with the lancet separation switch 182 or combined with the bar 181, as in an exemplary embodiment of the present invention, and is disposed in the internal space between the bar 181 and the housing 130 in the longitudinal direction of the bar 181. Further, the elastic member 183 may be compressed in the movement direction of the lancet separation switch 182 or may be returned by the elastic force of the elastic member 183 after compressed.

Wrinkles 182b are formed perpendicular to the longitudinal direction on the outer side of the lancet separation switch 182 and increase the friction force between the user's fingers and the surface, such that the lancet separation switch 128 can be easily longitudinally moved.

As shown in FIG. 3, when the lancet separation switch 182 is pushed with the lancet 10 spaced at a predetermined distance from the bar 182, the bar 181 is moved to the lancet 10 through the separation hole 119, such that the lancet 10 can be separated from the lancet holder 110.

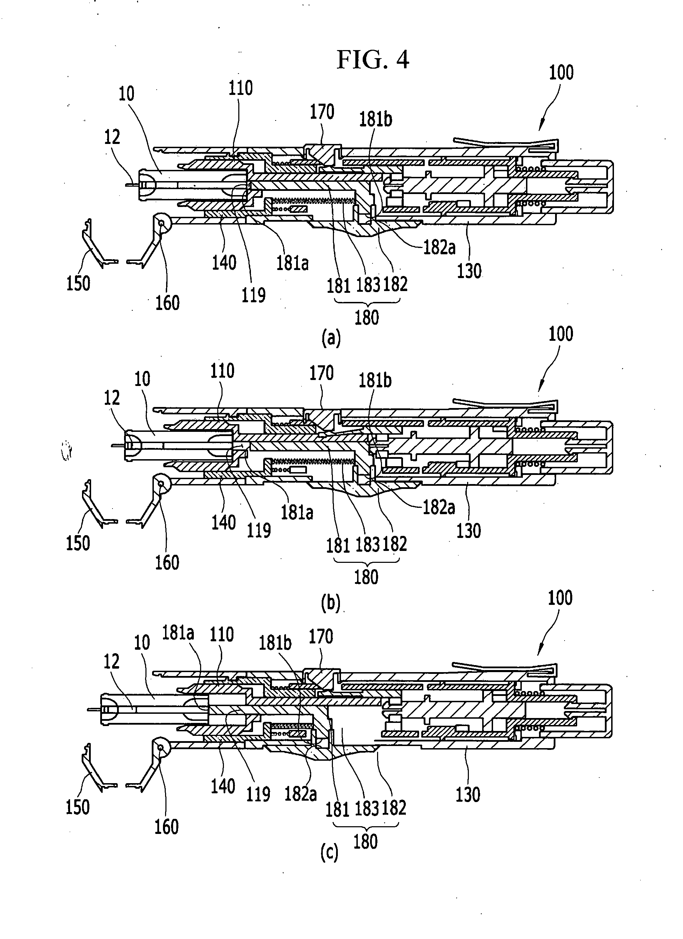

How to use the lancet separation mechanism 180 is described below in more detail with reference to FIG. 4.

FIG. 4 is a status view for illustrating how to use a lancet separation mechanism of the lancet-separable lancet device according to an exemplary embodiment of the present invention.

First, the cover 150 is opened and the lancet 10 is mounted on the lancet holder 110 (a).

Thereafter, the cover 150 is closed and the under-skin penetration depth of the lancet needle 12 is adjusted, and then the operating mechanism 120 is operated by pressing the operation switch 170, thereby taking blood. The detailed description of this step is not provided.

Next, it is required to separate the lancet 10 from the lancet device 100, in which the lancet separation switch 180 is pushed to the lancet 10 so that the bar 181 is inserted into the lancet holder 110 through the separation hole 119. Accordingly, the lancet 10 is separated from the lancet holder 110 by the bar 181 and the elastic member 183 is compressed in the movement direction of the lancet separation switch 182 and the bar 181 (b).

When the lancet separation switch 182 is released, after the lancet 10 is separated, the elastic member 183 returns to the initial status by the restoring force, and accordingly, the lancet separation switch 182 and the bar 181 are also moved in the direction opposite to the compression direction of the elastic member 183, returning to the initial position (c).

According to the structure and the operation way of the lancet separation mechanism 180, since the lancet separation switch 182 is used while the lancet 10 that finished taking blood is separated from the lancet device 100, it is possible to minimize the possibility of sticking the skin of the subject or people around with the lancet needle 12.

That is, according to the existing products, since it is required to pull out the lancet 10 to separate it from the lancet device 100 after taking blood, there is a problem in that the lancet 10 may spring in separating or a user may be pricked by the lancet needle 12; however, in the present invention, a subject can not only easily use the lancet separation switch 182, but stably separate the lancet 10.

Therefore, it is possible to minimize infection by viruses due to the reason that the examiner's fingers are pricked or touched by the lancet needle 12 used for taking blood.

The above description is an example of the spirit of the present invention and may be changed and modified in various ways by those skilled in the art without departing from the scope of the present invention. Therefore, the exemplary embodiments described herein are not for limiting the spirit of the present invention, but for explaining the present invention and the scope of the present invention is not limited to the exemplary embodiments. The protective range of the present invention should be construed by claims and the equivalents should be construed as being included in the scope of the present invention.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

## Claims

1. A lancet-separable lancet device, comprising: a lancet holder (110) having a lancet seat (111) to mount a lancet (10) at one end and a separation hole (119) at the other end; and a lancet separation mechanism (180) inserted into the separation hole (119) of the lancet holder (110) and separating the lancet (10) from the lancet holder (110) by pressing the lancet (10), wherein the lancet separation mechanism 1 (180) includes: a bar (181) having one end (181a) that is inserted into the lancet holder through the separation hole (119) of the lancet holder (110), a lancet separation switch (182) coupled to the other end (181b) of the bar (181) and moving one end of the bar (181) to the lancet 10 and an elastic member (183) combined with the bar (181) or the lancet separation switch (182) and returning the bar (181) inserted in the lancet holder (110) to the initial position, characterized that the bar (181) is formed in an L-shape and has a step at the other end (181b) and the lancet separation switch (182) has a fitting recess (182a) therein in which the other end of the bar (181) is fitted.

2. The device of claim 1, wherein the lancet separation switch (182) is fitted longitudinally in a coupling hole (139) formed on the outer side of the housing (130) and movable in the longitudinal direction.

3. The device of claim 1, wherein wrinkles (182b) perpendicular to the longitudinal direction are formed on the outer side of the lancet separation switch (182) which is fitted on the outer side of the housing (130).

## Patentansprüche

1. Eine lanzettentrennbare Lanzettenvorrichtung, die Folgendes umfasst:
einen Lanzettenhalter (110) mit einem Lanzettensitz (111) zur Montage einer Lanzette (10) an einem Ende und ein Trennloch (119) am anderen Ende; und einen Lanzetten-Trennmechanismus (180), der in das Trennloch (119) des Lanzettenhalters (110) eingesetzt ist, der die Lanzette (10) vom Lanzettenhalter (110) durch Drücken der Lanzette (10) trennt, wobei der Lanzetten-Trennmechanismus (180) Folgendes einschließt: einen Stab (181) mit einem Ende (181a), das durch das Trennloch (119) des Lanzettenhalters (110) in den Lanzettenhalter eingesetzt wird, einen Lanzetten-Trennschalter (182), gekoppelt mit dem anderen Ende (181b) des Stabs (181), der ein Ende des Stabs (181) zur Lanzette (10) bewegt, und ein elastisches Glied- (183), verbunden mit dem Stab (181) oder dem Lanzetten-Trennschalter (182), das den in den Lanzettenhalter (110) eingesetzten Stab (181) in die Ausgangsposition zurückstellt, **dadurch gekennzeichnet, dass**
der Stab (181) eine L-Form und eine Stufe am anderen Ende (181b) hat und der Lanzetten-Trennschalter (182) eine passende Vertiefung (182a) darin hat, in die das andere Ende des Stabs (181) eingesetzt ist.

2. Die Vorrichtung gemäß Anspruch 1, wobei der Lanzetten-Trennschalter (182) in Längsrichtung in ein Verbindungsloch (139) eingepasst ist, das an der Außenseite des Gehäuses (130) geformt und in Längsrichtung beweglich ist.

3. Die Vorrichtung gemäß Anspruch 1, wobei Falten (182b), senkrecht zur Längsrichtung, an der Außenseite des Lanzetten-Trennschalters (182) geformt sind, der an der Außenseite des Gehäuses (130) angebracht ist.

## Revendications

1. Dispositif à lancette à lancette séparable, comprenant: un support de lancette (110) présentant un siège de lancette (111) pour monter une lancette (10) à une extrémité et un trou de séparation (119) à l'autre extrémité; et un mécanisme de séparation de lancette (180) inséré dans le trou de séparation (119) du support de lancette (110) et séparant la lancette (10) du support de lancette (110) en faisant pression sur la lancette (10), dans lequel le mécanisme de séparation de lancette (180) comprend: une barre (181) ayant une extrémité (181a) qui est insérée dans le support de lancette à travers le trou de séparation (119) du support de lancette (110), un commutateur de séparation de lancette (182) couplé à l'autre extrémité (181b) de la barre (181) et déplaçant une extrémité de la barre (181) vers la lancette (10), et un élément élastique (183) combiné avec la barre (181) ou le commutateur de séparation de lancette (182) et faisant revenir la barre (181) insérée dans le support de lancette (110) à la position initiale, **caractérisé en ce que**
la barre (181) est formée en forme de L et présente un gradin à l'autre extrémité (181b) et le commutateur de séparation de lancette (182) présente un évidement d'ajustement (182a) dans lequel l'autre extrémité de la barre (181) est adaptée.

2. Dispositif selon la revendication 1, dans lequel le commutateur de séparation de lancette (182) est monté longitudinalement dans un trou de couplage (139) formé sur le côté extérieur du boîtier (130) et mobile dans la direction longitudinale.

3. Dispositif selon la revendication 1, dans lequel des sillons (182b) perpendiculaires à la direction longitudinale sont formés sur le côté extérieur du commutateur de séparation de lancette (182) qui est monté sur le côté extérieur du boîtier (130).
